# EUROPÄISCHE PATENTSCHRIFT

(11) **EP 1 948 181 B1**
(45) Veröffentlichungstag und Bekanntmachung des Hinweises auf die Patenterteilung: **07.08.2013**
(21) Anmeldenummer: 06828931.3
(22) Anmeldetag: 07.11.2006
(51) Int. Cl.: A61K 31/519, A61P 15/18, A61K 31/567, A61K 31/57

(54) **DARREICHUNGSFORM ZUR HORMONALEN KONTRAZEPTION**
DOSAGE FORM FOR HORMONAL CONTRACEPTION
FORME D´ADMINISTRATION D´UNE CONTRACEPTION HORMONALE

(30) Priorität: 09.11.2005 DE 102005053771
(43) Veröffentlichungstag der Anmeldung: 30.07.2008
(73) Patentinhaber: Richter Gedeon Nyrt., 1103 Budapest (HU)
(72) Erfinder: SCHRAMM, Georg, 52224 Stolberg (DE); PÂQUES, Eric-Paul, 52076 Aachen (DE)
(74) Vertreter: Kutzenberger, Helga
(86) Internationale Anmeldenummer: PCT/EP2006/010631
(87) Internationale Veröffentlichungsnummer: WO 2007/054258

(56) Entgegenhaltungen:
- WO-A-2005/115349
- WO-A2-99/53910
- "FORTIFYING ORAL CONTRACEPTIVES WITH FOLIC ACID" AWHONN LIFELINES / ASSOCIATION OF WOMEN'S HEALTH, OBSTETRIC AND NEONATAL NURSES, XX, US, Bd. 8, Nr. 1, Februar 2004 (2004-02), Seiten 12-13, XP009052123 ISSN: 1091-5923

## Beschreibung

Die vorliegende Erfindung betrifft eine Darreichungsform zur hormonalen Kontrazeption bestehend aus einer bestimmten Anzahl von hormonhaltigen Tages- Einheiten A und von 7-3 hormonhaltigen Tages-Einheiten B, deren Hormonkomponente jeweils nur aus einem Gestagen besteht, zur ununterbrochenen täglichen, oralen Verabreichung der hormonhaltigen Tages-Einheiten A, unmittelbar gefolgt von einer ununterbrochenen täglichen, oralen Verabreichung der hormonhaltigen Tages-Einheiten B an Frauen, dadurch gekennzeichnet, dass die hormonhaltigen Tages-Einheiten A jeweils Folsäure in einer Tagesmenge bis zu höchstens 200 µg und die hormonhaltigen Tages-Einheiten B jeweils Folsäure in einer Tagesmenge von mehr als 200 µg bis zu der bei Frauen maximal zulässigen Menge an Folsäure enthalten.

Hormonale Kontrazeptiva auf Basis eines Gestagens als Hormonkomponente sind als Gestagenpille bekannt.

Es wird vermutet, dass eine lang andauernde Einnahme dieser hormonalen Kontrazeptiva auf Basis von Gestagenen zu einem Mangel an Folsäure führen kann. Dieser Mangel kann beispielsweise zu cardiovaskulären Erkrankungen führen.

Außerdem ist bekannt, dass, sofern es innerhalb einer kurzer Zeit nach Abbruch der Einnahme solcher hormonaler Kontrazeptiva zu einer Schwangerschaft kommt, die Gefahr besteht, dass der Mangel an Folsäure bei dem Embryo zu Neuralrohrdefekten führen kann. Da das Neuralrohr in den ersten Wochen der Schwangerschaft entwickelt wird, ist es besonders vorteilhaft, eine präkonzeptionelle Einnahme von Folsäure zu gewährleisten.

Wenn daher wegen eines Kinderwunsches die Einnahme der Gestagen-Pille unterbrochen wird und es bereits im ersten Zyklus nach dem Absetzen der Gestagen-Pille zu einer Schwangerschaft kommt, ist die Gewährleistung einer entsprechend hohen Menge an Folsäure für die Zeit direkt nach dem Absetzen der Gestagen-Pille besonders wichtig.

Es besteht daher ein Bedarf, hormonale Kontrazeptiva auf Basis von einem Gestagen als einzige Hormonkomponente mit Folsäure in einer solchen Art und Weise auszurüsten, dass die zugesetzte Menge an Folsäure den unterschiedlichen Bedürfnissen über die Zeit während eines Einnahmezyklus und danach angepasst ist.

Aus WO 99/53910 ist bereits die Kombination von hormonalen Kontrazeptiva und Folsäure bekannt. Die Folsäuremenge pro hormonaler Tagesdosis ist nur entsprechend dem unterschiedlichen Bedarf an Folsäure entsprechend dem fortschreitenden Alter einer Frau angepasst. Es wird aber nicht der unterschiedliche Bedarf an Folsäure über den Einnahmezyklus eines Kontrazeptivums berücksichtigt.

Daher war es Aufgabe der vorliegenden Erfindung, eine Darreichungsform zur hormonalen Kontrazeption auf Basis nur eines Gestagens als Hormonkomponente zur Verfügung zu stellen, die den unterschiedlichen Bedarf an Folsäure während des hormonalen Einnahmezyklus berücksichtigt.

Diese Aufgabe wurde durch die Bereitstellung der erfindungsgemäßen Darreichungsform zur hormonalen Kontrazeption bestehend aus einer bestimmten Anzahl von hormonhaltigen Tages-Einheiten A und von 7-3 hormonhaltigen Tages-Einheiten B, deren Hormonkomponente jeweils nur aus einem Gestagen besteht, zur ununterbrochenen täglichen, oralen Verabreichung der hormonhaltigen Tages-Einheiten A, unmittelbar gefolgt von einer ununterbrochenen täglichen, oralen Verabreichung der hormonhaltigen Tages-Einheiten B an Frauen, dadurch gekennzeichnet, dass die hormonhaltigen Tages-Einheiten A jeweils Folsäure in einer Tagesmenge bis zu höchstens 200 µg und die hormonhaltigen Tages-Einheiten B jeweils Folsäure in einer Tagesmenge größer als 200 µg bis zu der bei Frauen maximal zulässigen Menge an Folsäure enthalten, gelöst.

Frauen im gebärfähigen Alter haben einen täglichen Bedarf an Folsäure, der durch eine gesunde Ernährung ausreichend gedeckt werden kann. Eine lang andauernde Einnahme von hormonalen Kontrazeptiva enthaltend Gestagene kann zu einem zusätzlichen Bedarf an Folsäure führen, der ebenfalls durch eine gesunde Ernährung gedeckt werden kann. Allerdings ist dafür eine tägliche Gabe der bei Frauen minimal wirksamen Tagesmenge an Folsäure empfehlenswert.

Dementsprechend können die hormonhaltigen Tages-Einheiten A der erfindungsgemäßen Darreichungsform jeweils eine Tagesmenge an Folsäure aufweisen, die diese minimal wirksame Tagesmenge an Folsäure mit umfasst. Vorzugsweise enthalten die hormonhaltigen Tages-Einheiten A der erfindungsgemäßen Darreichungsform 5 bis 200 µg Folsäure.

Um bei einem angestrebten Kinderwunsch möglichst rasch die notwendige Menge an Folsäure bzw. den erhöhten Bedarf an Folsäure zumindest bei Beginn einer Schwangerschaft zu gewährleisten, und dabei einer mögliche Schädigung des Embryos durch Folsäuremangel vorzubeugen, enthalten die hormonhaltigen Tages-Einheiten B der erfindungsgemäßen Darreichungsform Folsäure in einer Menge von mehr als 200 µg bis zur maximal zulässigen Tagesmenge an Folsäure für Frauen, vorzugsweise bis zu 5 mg Folsäure pro Tages-Einheit, besonders bevorzugt von mehr als 200 µg bis 5 mg Folsäure, ganz besonders bevorzugt bis zu der bei fertilen Frauen maximal zulässige Tagesmenge an Folsäure.

Durch den Zusatz von Folsäure zu den letzten 7-3 hormonhaltigen Tages-Einheiten eines Einnahmezyklus, nämlich den hormonhaltigen Tages-Einheiten B der erfindungsgemäßen Darreichungsform in Mengen bis zur maximal für fertile Frauen zulässigen Menge kann schon während der Einnahme dieser hormonhaltigen Tages-Einheiten B die Folsäurekonzentration im weiblichen Körper soweit erhöht werden, dass gegebenenfalls bei einem Absetzen der Einnahme des Gestagen-Kontrazeptivums und einer darauffolgenden Schwangerschaft möglichst frühzeitig der zu Beginn einer Schwangerschaft erhöhte Folsäurebedarf im weiblichen Körper gewährleistet ist.

Vorzugsweise enthalten die hormonhaltigen Tages-Einheiten A der erfindungsgemäßen Darreichungsform jeweils dieselbe Menge Folsäure. Dies gilt auch für die hormonhaltigen Tages-Einheiten B, die ebenfalls jeweils dieselbe, gegenüber den hormonhaltigen Tages-Einheiten A aber erhöhte Menge Folsäure aufweisen.

Die Folsäure kann in der erfindungsgemäßen Darreichungsform auch als pharmazeutisch unbedenkliches Salz, vorzugsweise als Natrium-, Kalium- oder Magnesium-Salz oder als ein entsprechendes Derivat vorliegen.

Als Derivate der Folsäure eignen sich Mono- oder Diester, wobei bei Diestern eine unterschiedliche oder identische Veresterung vorliegen kann. Vorzugsweise eignet sich als Alkoholrest eine kurzkettige Alkylgruppe mit C₁-C₈, wie Methyl, Ethyl, Propyl oder Butyl, eine verzweigte, kurzkettige Alkylgruppe mit C₃-C₈, wie Isopropyl, Isobutyl oder sec. Butyl, eine Cycloalkyl-Gruppe, wie Cyclopentyl oder Cyclohexyl, eine ArylGruppe, wie Phenyl oder substituiertes Phenyl mit 1-2 Substituenten, z. B. mit einer niedrigen Alkyl- oder Halogen-Alkoxyl-Gruppe, oder einer Arylalkyl-Gruppe mit einem C₁-C₈ Alkylrest und eine Arylgruppe, wie Phenyl oder substituiertes Phenyl.

Darüber hinaus können die hormonhaltigen Tages-Einheiten B und gegebenenfalls die hormonhaltigen Tages-Einheiten A zusätzlich zur Folsäure weitere Vitamine oder Mineralien enthalten. Bevorzugt sind keine weiteren Zusätze dieser Art.

Die Anzahl der Gestagen-haltigen Tages-Einheiten einer erfindungsgemäßen Darreichungsform kann einem natürlichen, monatlichen, weiblichen MenstruationsZyklus entsprechen. In diesem Fall enthält die erfindungsgemäße Darreichungsform 21 bis 25 hormonhaltige Tages-Einheiten A zur ununterbrochenen, oralen, täglichen Einname und 7 bis 3 hormonhaltigen Tages-Einheiten B zur unmittelbar anschließenden ununterbrochenen, täglichen Einnahme.

Es ist aber auch möglich, dass die Gesamtzahl der hormonhaltigen Tages-Einheiten A größer ist als es einem natürlichen, weiblichen Monatszyklus entspricht, so dass eine erfindungsgemäße Darreichungsform hormonhaltige Tages-Einheiten A für eine ununterbrochene Einnahme bis zu 2 Jahren, vorzugsweise bis zu 1 Jahr, und für eine unmittelbar anschließende ununterbrochene Einnahme 7 bis 3 hormonhaltigen Tages-Einheiten B enthalten kann. Es ist aber auch möglich, dass die erfindungsgemäße Darreichungsform 42 bis 52 bzw. 77 bis 193 hormonhaltige Tages-Einheiten A und zur unmittelbar anschließenden Einnahme die letzten 7 bis 3 hormonhaltigen Tages-Einheiten, nämlich der Tages-Einheiten B, aufweisen kann.

Die hormonhaltigen Tages-Einheiten der erfindungsgemäßen Darreichungsform enthalten jeweils denselben Gehalt eines kontrazeptiv wirkenden Gestagens als einzige Hormonkomponente. Die erfindungsgemäß zum Einsatz kommenden Gestagen-haltigen Tage-Einheiten A bzw. B enthalten vorzugsweise jeweils ein Gestagen, vorzugsweise dasselbe Gestagen in einer Menge, die mindestens der Ovulationshemmdosis entspricht. Besonders bevorzugt liegt die Menge des Gestagens bis zu 150% über der Ovulationshemmdosis.

Für die hormonhaltigen Tages-Einheiten A bzw. B der erfindungsgemäßen Darreichungsform eignen sich Gestagene, die vorzugsweise aus der Gruppe umfassend Norethisteron, Norethisteronacetat, Norethisteronenantat, Norgestimat, Norgestrel, Levonorgestrel, Gestoden, Hydroxyprogesteroncaproat, Medroxyprogesteronacetat, Megestrolacetat, Chlormadinonacetat, Lynestrenol, Cyproteronacetat, Drospirenon, Dienogest, Desogestrel, Progesteron, Dydrogesteron, Medrogeston, Ethynodiol,-diacetat, Promegeston, Nomegestrolacetat, Trimegeston, Etonogestrel, Norelgestromin, Norethynodrel und Tibolon ausgewählt sind.

Die Hormone werden vorzugsweise in den nachstehend angegebenen Mengen verwendet:

| | |
|---|---|
| Norethisteron, -acetat | 0,5 bis 2,0 mg |
| Norgestimat | 0,1 bis 0,25 mg |
| Norgestrel | 0,3 bis 2,0 mg |
| Levonorgestrel | 0,06 bis 0,15 mg |
| Gestoden | 0,03 bis 0,12 mg |
| Hydroxyprogesteroncaproat | 10 bis 800 mg |
| Medroxyprogesteronacetat | 5,0 bis 40 mg |
| Megestrolacetat | 1,0 bis 10 mg |
| Chlormadinonacetat | 1,5 bis 10 mg |
| Lynestrenol | 0,8 bis 3 mg |
| Cyproteronacetat | 1,0 bis 10 mg |
| Drospirenon | 1,0 bis 10 mg |
| Dienogest | 1,0 bis 10 mg |
| Desogestrel | 0,06 bis 0,20 mg |
| Progesteron | 400 bis 2000 mg |
| Dydrogesteron | 5 bis 50 mg |
| Medrogeston | 2 bis 30 mg |
| Ethynodiol, -diacetat | 0,4 bis 3 mg |
| Promegeston | 0,5 bis 10 mg |
| Nomegestrolacetat | 0,5 bis 10 mg |
| Trimegeston | 0,1 bis 10 mg |
| Etonogestrel | 0,1 bis 2 mg |
| Norelgestromin | 0,1 bis 2 mg |
| Norethynodrel | 0,3 bis 3 mg |
| Tibolon | 1 bis 10 mg |

Vorzugsweise weisen sowohl die Gestagen-haltigen Tages-Einheiten A als auch B 1,5 bis 5 mg Chlormadinonacetat auf, wobei mindestens 21 Tages-Einheiten A, vorzugsweise 21 bis 25 Tages-Einheiten A und 3 bis 7 Gestagen-haltige Tages-Einheiten B in der erfindungsgemäßen Darreichungsform vorliegen. Die erfindungsgemäße Darreichungsform kann aber auch Gestagen-haltige Tages-Einheiten A für mehrere Jahre, vorzugsweise 42 bis 365 Gestagen-haltige Tages-Einheiten A umfassen, wobei sich an deren ununterbrochene Einnahmezeit unmittelbar eine ununterbrochene Einnahme von 7 bis 3 Gestagen-haltigen Tages-Einheiten B mit jeweils der erfindungsgemäß angegebenen, erhöhten Menge Folsäure anschließt.

Die erfindungsgemäße Darreichungsform umfasst die Gestagen-haltigen Tages-Einheiten A und B, vorzugsweise in Form von Tabletten, die im Blister verpackt sind, die auch vorzugsweise eine Einnahme-Markierung aufweisen. Dies ist insbesondere von Vorteil, wenn die erfindungsgemäße Darreichungsform als ein Kontrazeptivum entsprechend dem weiblichen Menstruationszyklus vorgesehen ist.

Die erfindungsgemäße Darreichungsform kann auch ein Bestandteil eines Kits sein, wobei das erfindungsgemäße Kit mehrere der erfindungsgemäßen Darreichungsformen umfassen kann, insbesondere wenn eine Darreichungsform nur einen monatlichen, weiblichen Menstruationszyklus umfasst. So kann das Kit auch mindestens 13 erfindungsgemäße Darreichungsformen entsprechend dem weiblichen Menstruationszyklus von 28 Tagen umfassen, so dass nach jeder Beendigung eines solchen Einnahmezyklus von 28 Tagen bei einem Kinderwunsch die weitere Einnahme gestoppt werden kann und eine bereits erhöhte Folsäurekonzentration für eine mögliche Schwangerschaft gewährleistet ist. Gegebenenfalls wird von dem Kit auch noch ein Kalender bzw. ein Kalenderbuch mit umfasst.

### Beispiele

### Beispiel 1:

### Zusammensetzung

| | A) | B) |
|---|---|---|
| | Pro Tablette | Pro Tablette |
| Chlormadinonacetat | 2,000 mg | 2,000 mg |
| Natriumfolat | 0,050 mg | 3,000 mg |
| Povidon K30 | 3,000 mg | 3,000 mg |
| Lactose | 31,930 mg | 31,000 mg |
| Maisstärke | 12,000 mg | 12,000 mg |
| Magnesiumstearat | 0,500 mg | 0,500 mg |
| hochdisperses Siliciumdioxid | 0,500 mg | 0,500 mg |

| | | |
|---|---|---|
| A) Povidon K 30 (Polyvinylpyrrolidon) und das Natriumsalz der Folsäure wurden in 600 ml Ethanol gelöst. Chlormadinonacetat (Partikelgröße 90% < 50 µm), Lactose und Maisstärke wurden in einem Mischer/Granulierer (Diosna P25) 5 min gemischt und anschließend mit der ethanolischen EE/PVP Lösung durchfeuchtet und gemischt. Die feuchte Masse wurde durch ein 3 mm Sieb getrieben und in einem Vakuumtrockenschrank getrocknet. Das trockene Granulat wurde durch ein 0,6 mm Sieb desagglomeriert, mit Magnesiumstearat und hochdispersem Siliciumdioxid gemischt und auf einer Tablettenpresse mit 5 mm Stempeln zu Tabletten mit einem Gewicht von 50 mg als Tageseinheiten A gepresst. B) Wie unter A) angegeben, wurden ebenso folsäurehaltige Tabletten als Tageseinheiten B mit einem Gewicht von 50 mg hergestellt, wobei das Natriumsalz der Folsäure in 600 ml wässrigem Ethanol gelöst wurde. | | |

Die Tabletten A bzw. B wurden jeweils mit einem Lack auf Basis Methylhydroxypropylcellulose überzogen (z. B. Opadry YS-1-2184 vom Hersteller Colorcon); Überzugsmasse 2 mg pro Tablette.

Zur Herstellung einer erfindungsgemäßen, kontrazeptiven Darreichungsform wurden 21 hormonhaltige Tabletten A und 7 hormonhaltige Tabletten B als 28 Tages-Einheiten zu einem Blister verpackt.

## Patentansprüche

1. Eine Darreichungsform zur hormonalen Kontrazeption bestehend aus einer bestimmten Anzahl von hormonhaltigen Tages-Einheiten A und von 7-3 hormonhaltigen Tages-Einheiten B, deren Hormonkomponente jeweils nur aus einem Gestagen besteht, zur ununterbrochenen täglichen, oralen Verabreichung der hormonhaltigen Tages-Einheiten A, unmittelbar gefolgt von einer ununterbrochenen täglichen, oralen Verabreichung der hormonhaltigen Tages-Einheiten B an Frauen, **dadurch gekennzeichnet, dass** die hormonhaltigen Tages-Einheiten A jeweils Folsäure in einer Tagesmenge von 5 bis 200 µg und die hormonhaltigen Tages-Einheiten B jeweils Folsäure in einer Tagesmenge größer als 200 µg bis zu der bei Frauen maximal zulässigen Menge an Folsäure enthalten.

2. Eine Darreichungsform nach Anspruch 1, **dadurch gekennzeichnet, dass** die hormonhaltigen Tages-Einheiten B jeweils mehr als 200 µg bis zu 5 mg Folsäure enthalten.

3. Eine Darreichungsform nach Anspruch 1 oder 2, **dadurch gekennzeichnet, dass** die hormonhaltigen Tages-Einheiten A jeweils dieselbe Menge Folsäure und die hormonhaltigen Tages-Einheiten B ebenfalls jeweils dieselbe Menge Folsäure enthalten.

4. Eine Darreichungsform nach einem der Ansprüche 1 bis 3, **dadurch gekennzeichnet, dass** sie wenigstens 21 hormonhaltige Tages-Einheiten A und 7 bis 3 hormonhaltige Tages-Einheiten B aufweist.

5. Eine Darreichungsform nach Anspruch 4, **dadurch gekennzeichnet, dass** deren maximale Anzahl an hormonhaltigen Tages-Einheiten A einer ununterbrochenen Verabreichung für mehrere Jahre, vorzugsweise bis zu 730, besonders bevorzugt bis zu 365 hormonhaltige Tages-Einheiten A und 7 bis 3 hormonhaltige Tages-Einheiten B aufweist.

6. Eine Darreichungsform nach Anspruch 4, **dadurch gekennzeichnet, dass** sie 21 bis 25 hormonhaltige Tages-Einheiten A und 7 bis 3 hormonhaltige Tages-Einheiten B oder 42 bis 52 hormonhaltige Tages-Einheiten A und 7 bis 3 hormonhaltige Tages-Einheiten B oder 77 bis 193 hormonhaltige Tages-Einheiten A und 7 bis 3 hormonhaltige Tages-Einheiten B aufweist.

7. Eine Darreichungsform nach einem der Ansprüche 1 bis 6, **dadurch gekennzeichnet, dass** sowohl die hormonhaltigen Tages-Einheiten A als auch B jeweils einen Gestagen-Gehalt aufweisen, der mindestens der Ovulationshemmdosis des Gestagens entspricht.

8. Eine Darreichungsform nach Anspruch 7, **dadurch gekennzeichnet, dass** der Gestagen-Gehalt bis zu 150% über der Ovulationshemmdosis des Gestagens liegt.

9. Eine Darreichungsform nach Anspruch 7 oder 8, **dadurch gekennzeichnet, dass** sowohl die hormonhaltigen Tages-Einheiten A als auch die hormonhaltigen Tages-Einheiten B jeweils dieselbe Menge Gestagen aufweisen.

10. Eine Darreichungsform nach einem der Ansprüche 1 bis 9, **dadurch gekennzeichnet, dass** eine hormonhaltige Tages-Einheit A bzw. B ein Gestagen aus der Gruppe umfassend Norethisteron, Norethisteronacetat, Norethisteronenantat, Norgestimat, Norgestrel, Levonorgestrel, Gestoden, Hydroxyprogesteroncaproat, Medroxyprogesteronacetat, Megestrolacetat, Chlormadinonacetat, Lynestrenol, Cyproteronacetat, Drospirenon, Dienogest, Desogestrel, Progesteron, Dydrogesteron, Medrogeston, Ethynodiol bzw. - diacetat, Promegeston, Nomegestrolacetat, Trimegeston, Etonogestrel, Norelgestromin, Norethynodrel und Tibolon enthält.

11. Eine Darreichungsform nach Anspruch 10, **dadurch gekennzeichnet**, das in einer hormonhaltige Tages-Einheit A bzw. B das Gestagen jeweils in einer Menge von
| | |
|---|---|
| 0,5 bis 2,0 mg | Norethisteron, -acetat |
| 0,1 bis 0,25 mg | Norgestimat |
| 0,3 bis 2,0 mg | Norgestrel |
| 0,06 bis 0,15 mg | Levonorgestrel |
| 0,03 bis 0,12 mg | Gestoden |
| 10 bis 800 mg | Hydroxyprogesteroncaproat |
| 5,0 bis 40 mg | Medroxyprogesteronacetat |
| 1,0 bis 10 mg | Megestrolacetat |
| 1,5 bis 10 mg | Chlormadinonacetat |
| 0,8 bis 3 mg | Lynestrenol |
| 1,0 bis 10 mg | Cyproteronacetat |
| 1,0 bis 10 mg | Drospirenon |
| 1,0 bis 10 mg | Dienogest |
| 400 bis 2000 mg | Progesteron |
| 5 bis 50 mg | Dydrogesteron |
| 0,06 bis 0,20 mg | Desogestrel |
| 2 bis 30 mg | Medrogeston |
| 0,4 bis 3 mg | Ethynodiol, -diacetat |
| 0,5 bis 10 mg | Promegeston |
| 0,5 bis 10 mg | Nomegestrolacetat |
| 0,1 bis 10 mg | Trimegeston |
| 0,1 bis 2 mg | Etonogestrel |
| 0,1 bis 2 mg | Norelgestromin |
| 0,3 bis 3 mg | Norethynodrel |
| 1 bis 10 mg | Tibolon |
vorliegt.

12. Eine Darreichungsform nach einem der Ansprüche 1 bis 11, **dadurch gekennzeichnet, dass** die Tages-Einheiten A bzw. B als Tabletten vorliegen.

13. Ein Kit enthaltend eine oder mehrere Darreichungsformen zur hormonalen Kontrazeption nach einem der Ansprüche 1 bis 12.

## Claims

1. A dosage form for hormonal contraception consisting of a specific number of hormone-containing daily units A and of 7-3 hormone-containing daily units B, the hormone component of which in each case consists solely of a gestagen, for uninterrupted daily, oral administration of the hormone-containing daily units A, immediately followed by uninterrupted daily, oral administration of the hormone-containing daily units B to women, **characterised in that** the hormone-containing daily units A in each case contain folic acid in a daily amount of 5 to 200 µg and the hormone-containing daily units B in each case contain folic acid in a daily amount of more than 200 µg up to the maximum permissible amount of folic acid for women.

2. A dosage form according to claim 1, **characterised in that** the hormone-containing daily units B each contain more than 200 µg and up to 5 mg of folic acid.

3. A dosage form according to claim 1 or claim 2, **characterised in that** the hormone-containing daily units A each contain the same amount of folic acid and the hormone-containing daily units B likewise each contain the same amount of folic acid.

4. A dosage form according to one of claims 1 to 3, **characterised in that** it comprises at least 21 hormone-containing daily units A and 7 to 3 hormone-containing daily units B.

5. A dosage form according to claim 4, **characterised in that** its maximum number of hormone-containing daily units A is sufficient for uninterrupted administration for a plurality of years, preferably up to 730, preferably up to 365, hormone-containing daily units A and 7 to 3 hormone-containing daily units B.

6. A dosage form according to claim 4, **characterised in that** it comprises 21 to 25 hormone-containing daily units A and 7 to 3 hormone-containing daily units B or 42 to 52 hormone-containing daily units A and 7 to 3 hormone-containing daily units B or 77 to 193 hormone-containing daily units A and 7 to 3 hormone-containing daily units B.

7. A dosage form according to one of claims 1 to 6, **characterised in that** both the hormone-containing daily units A and B in each case have a gestagen content which at least corresponds to the ovulation inhibition dose of the gestagen.

8. A dosage form according to claim 7, **characterised in that** the gestagen content is up to 150% above the ovulation inhibition dose of the gestagen.

9. A dosage form according to claim 7 or claim 8, **characterised in that** both the hormone-containing daily units A and the hormone-containing daily units B in each case comprise the same amount of gestagen.

10. A dosage form according to one of claims 1 to 9, **characterised in that** a hormone-containing daily unit A or B contains a gestagen from the group comprising norethisterone, norethisterone acetate, norethisterone enantate, norgestimate, norgestrel, levonorgestrel, gestodene, hydroxyprogesterone caproate, medroxyprogesterone acetate, megestrol acetate, chlormadinone acetate, lynestrenol, cyproterone acetate, drospirenone, dienogest, desogestrel, progesterone, dydrogesterone, medrogestone, ethynodiol or ethynodiol diacetate, promegestone, nomegestrol acetate, trimegestone, etonogestrel, norelgestromin, norethynodrel and tibolone.

11. A dosage form according to claim 10, **characterised in that** a hormone-containing daily unit A or B contains the gestagen in each case in an amount of
| | |
|---|---|
| 0.5 to 2.0 mg | norethisterone, norethisterone acetate |
| 0.1 to 0.25 mg | norgestimate |
| 0.3 to 2.0 mg | norgestrel |
| 0.06 to 0.15 mg | levonorgestrel |
| 0.03 to 0.12 mg | gestodene |
| 10 to 800 mg | hydroxyprogesterone caproate |
| 5.0 to 40 mg | medroxyprogesterone acetate |
| 1.0 to 10 mg | megestrol acetate |
| 1.5 to 10 mg | chlormadinone acetate |
| 0.8 to 3 mg | lynestrenol |
| 1.0 to 10 mg | cyproterone acetate |
| 1.0 to 10 mg | drospirenone |
| 1.0 to 10 mg | dienogest |
| 400 to 2000 mg | progesterone |
| 5 to 50 mg | dydrogesterone |
| 0.06 to 0.20 mg | desogestrel |
| 2 to 30 mg | medrogestone |
| 0.4 to 3 mg | ethynodiol, ethynodiol diacetate |
| 0.5 to 10 mg | promegestone |
| 0.5 to 10 mg | nomegestrol acetate |
| 0.1 to 10 mg | trimegestone |
| 0.1 to 2 mg | etonogestrel |
| 0.1 to 2 mg | norelgestromin |
| 0.3 to 3 mg | norethynodrel |
| 1 to 10 mg | tibolone. |

12. A dosage form according to one of claims 1 to 11, **characterised in that** the daily units A or B assume tablet form.

13. A kit containing at least one dosage form for hormonal contraception according to one of claims 1 to 12.

## Revendications

1. Forme d'administration pour la contraception hormonale constituée par un nombre déterminé d'unités journalières A contenant des hormones et par 7-3 unités journalières B contenant des hormones, dont le composant hormonal est à chaque fois constitué par seulement un gestagène, pour l'administration par voie orale, journalière, ininterrompue des unités journalières A contenant des hormones, suivie directement d'une administration par voie orale, journalière, ininterrompue des unités journalières B contenant des hormones, **caractérisée en ce que** les unités journalières A contenant des hormones contiennent à chaque fois de l'acide folique en une quantité journalière de 5 à 200 µg et les unités journalières B contenant des hormones contiennent à chaque fois de l'acide folique en une quantité journalière supérieure à 200 µg jusqu'à la quantité maximale d'acide folique autorisée pour les femmes.

2. Forme d'administration selon la revendication 1, **caractérisée en ce que** les unités journalières B contenant des hormones contiennent à chaque fois plus de 200 µg jusqu'à 5 mg d'acide folique.

3. Forme d'administration selon la revendication 1 ou 2, **caractérisée en ce que** les unités journalières A contenant des hormones contiennent à chaque fois la même quantité d'acide folique et les unités journalières B contenant des hormones contiennent également à chaque fois la même quantité acide folique.

4. Forme d'administration selon l'une quelconque des revendications 1 à 3, **caractérisée en ce qu'**elle présente au moins 21 unités journalières A contenant des hormones et 7 à 3 unités journalières B contenant des hormones.

5. Forme d'administration selon la revendication 4, **caractérisée en ce que** son nombre maximal d'unités journalières A contenant des hormones représente une administration ininterrompue pour plusieurs années, de préférence jusqu'à 730, de manière particulièrement préférée jusqu'à 365 unités journalières A contenant des hormones et 7 à 3 unités journalières B contenant des hormones.

6. Forme d'administration selon la revendication 4, **caractérisée en ce qu'**elle présente 21 à 25 unités journalières A contenant des hormones et 7 à 3 unités journalières B contenant des hormones ou 42 à 52 unités journalières A contenant des hormones et 7 à 3 unités journalières B contenant des hormones ou 77 à 193 unités journalières A contenant des hormones et 7 à 3 unités journalières B contenant des hormones.

7. Forme d'administration selon l'une quelconque des revendications 1 à 6, **caractérisée en ce que** tant les unités journalières A que les unités journalières B contenant des hormones présentent à chaque fois une teneur en gestagène qui correspond au moins à la dose d'inhibition de l'ovulation du gestagène.

8. Forme d'administration selon la revendication 7, **caractérisée en ce que** la teneur en gestagène est de jusqu'à 150% supérieure à la dose d'inhibition de l'ovulation du gestagène.

9. Forme d'administration selon la revendication 7 ou 8, **caractérisée en ce que** tant les unités journalières A contenant des hormones que les unités journalières B contenant des hormones présentent à chaque fois la même quantité de gestagène.

10. Forme d'administration selon l'une quelconque des revendications 1 à 9, **caractérisée en ce qu'**une unité journalière A ou, selon le cas, une unité journalière B contenant des hormones contient un gestagène du groupe comprenant la noréthistérone, l'acétate de noréthistérone, l'énanthate de noréthistérone, le norgestimate, le norgestrel, le lévonorgestrel, le gestodène, le caproate d'hydroxyprogestérone, l'acétate de médroxyprogestérone, l'acétate de mégestrol, l'acétate de chlormadinone, le lynestrénol, l'acétate de cyprotérone, le drospirénone, le diénogest, le désogestrel, la progestérone, la dydrogestérone, la médrogestone, l'éthynodiol ou, selon le cas, le diacétate d'éthynodiol, la promégestone, l'acétate de nomégestrol, la trimégestone, l'étonogestrel, la norelgestromine, le noréthynodrel et la tibolone.

11. Forme d'administration selon la revendication 10, **caractérisée en ce qu'**une unité journalière A ou, selon le cas, une unité journalière B contenant des hormones contient le gestadène en une quantité de 0,5 à 2,0 mg de noréthistérone, d'acétate de noréthistérone
| | |
|---|---|
| 0,1 à 0,25 mg | de norgestimate |
| 0,3 à 2,0 mg | de norgestrel |
| 0,06 à 0,15 mg | de lévonorgestrel |
| 0,03 à 0,12 mg | de gestodène |
| 10 à 800 mg | de caproate d'hydroxyprogestérone |
| 5,0 à 40 mg | d'acétate de médroxyprogestérone |
| 1,0 à 10 mg | d'acétate de mégestrol |
| 1,5 à 10 mg | d'acétate de chlormadinone |
| 0,8 à 3 mg | de lynestrénol |
| 1,0 à 10 mg | d'acétate de cyprotérone |
| 1,0 à 10 mg | de drospirénone |
| 1,0 à 10 mg | de diénogest |
| 400 à 2000 mg | de progestérone |
| 5 à 50 mg | de dydrogestérone |
| 0,06 à 0,20 mg | de désogestrel |
| 2 à 30 mg | de médrogestone |
| 0,4 à 3 mg | d'éthynodiol, de diacétate d'éthynodiol |
| 0,5 à 10 mg | de promégestone |
| 0,5 à 10 mg | d'acétate de nomégestrol |
| 0,1 à 10 mg | de trimégestone |
| 0,1 à 2 mg | d'étonogestrel |
| 0,1 à 2 mg | de norelgestromine |
| 0,3 à 3 mg | de noréthynodrel |
| 1 à 10 mg | de tibolone. |

12. Forme d'administration selon l'une quelconque des revendications 1 à 11, **caractérisée en ce que** les unités journalières A ou, selon le cas, les unités journalières B se trouvent sous forme de comprimés.

13. Kit contenant une ou plusieurs formes d'administration pour la contraception hormonale selon l'une quelconque des revendications 1 à 12.
